# EUROPÄISCHE PATENTSCHRIFT

(11) **EP 0 912 167 B1**
(45) Veröffentlichungstag und Bekanntmachung des Hinweises auf die Patenterteilung: **13.11.2002**
(21) Anmeldenummer: 97931773.2
(22) Anmeldetag: 04.07.1997
(51) Int. Cl.: A61K 9/16

(54) **ORAL APPLIZIERBARE GRANULATE VON HEXAHYDROPYRAZINDERIVATEN**
GRANULATES OF HEXAHYDROPYRAZINE DERIVATIVES WHICH CAN BE ADMINISTERED ORALLY
GRANULATS DE DERIVES D'HEXAHYDROPYRAZINE, ADMINISTRABLES PAR VOIE ORALE

(30) Priorität: 17.07.1996 DE 19628776
(43) Veröffentlichungstag der Anmeldung: 06.05.1999
(73) Patentinhaber: BAYER AG, 51368 Leverkusen (DE)
(72) Erfinder: KALBE, Jochen, D-42799 Leichlingen (DE); HOPKINS, Terence, Tamborine, QLD 4270 (AU)
(86) Internationale Anmeldenummer: EP9703537
(87) Internationale Veröffentlichungsnummer: WO98003157

(56) Entgegenhaltungen:
- EP-A- 0 252 407
- EP-A- 0 717 993
- US-A- 4 447 414
- CHEMICAL ABSTRACTS, vol. 123, no. 14, 2.Oktober 1995 Columbus, Ohio, US; abstract no. 179458, XP002044361 & CN 1 099 973 A (SICHUAN MEDICAL SCIENCE ACADEMY) 15.März 1995

## Beschreibung

Die vorliegende Erfindung betrifft die Herstellung oral applizierbarer Granulate von Hexahydropyrazinderivaten sowie Granulate, die nach diesem Verfahren hergestellt sind.

Hexahydropyrazinderivate wie z.B. Praziquantel und Epsiprantel sind bekannt (siehe US-P 4 001 411, EP-A 134 984). Durch den bitteren Geschmack der Wirkstoffe ist eine einfache orale Verabreichung an geschmacksempfindliche Tiere wie z.B. Katzen nicht ohne weiteres möglich. Die Wirkstoffe lassen sich auch nicht durch die üblichen Methoden der Geschmacksmarkierung wie Salzbildung mit Embonsäure oder Bindung an Ionenaustauscher in oral anwendbare Mittel überführen. Erfahrungen haben gezeigt, daß oral an Katzen verabreichbare Mittel dieser Wirkstoffe auch nicht durch Geschmacksmarkierung mit Aromastoffen oder unter Einsatz von verkapselten Formulierungen erhalten werden können.

Es ist bekannt, daß Hexahydropyrazinderivate wie z.B. Praziquantel durch Säulenchromatographie an chiralen Packungsmaterialien wie z.B. Cellulosetriacetat in ihre Enantiomere getrennt werden können (siehe Lim Beegim, Ching, Chibun, Industrial & Engineering Chemnistry Research, Vol 35, 1996, 169-175; Lim Beegim, Ching Chibun, Separations Technology, Vol 5, 1995, 213-228; Blaschke G, Journal of Liquid Chromatoraphy, Vol 9/2-3, 1986, 341-368). Diese Trennung kommt durch die unterschiedliche schwache Wechselwirkung zwischen der Säulenpackung und den Enantiomeren des Wirkstoffs zustande. Es gibt keine Hinweise darauf, daß auf diese Weise Wirkstoff-Trägerkomplexe erhalten werden können die geschmacksneutral sind.

EP-A 252 407 offenbart ein Verfahren zur kontinuierlichen Herstellung von sphärischen Granulaten. Beispielhaft ist unter anderem die Herstellung von Granulaten enthaltend einen Chinolon-Wirkstoff oder Praziquantel und als Bindemittel Polyvinylpyrrolidon oder kaltquellbare Maisstärke beschrieben. Zur Geschmacksmaskierung wird empfohlen die hergestellten Pellets mit einer Lackschicht zu überziehen. Granulate mit hydrophoben Trägerstoffen werden nicht beschrieben.

Es wurde nun gefunden, daß man oral applizierbare Formulierungen von Hexahydropyrazinderivaten erhält, indem man den Wirkstoff in Gegenwart geeigneter Lösungsmittel mit hydrophoben Trägerstoffen gegebenenfalls in Gegenwart von Hilfsstoffen vermischt und die erhaltene Mischung gegebenenfalls in weitere anwendungsfertige Formen überführt.

Nach dem erfindungsgemäßen Verfahren wird ein Granulat erhalten, das Hexahydropyrazinderivate enthält, das ohne Probleme auch Tieren oral verabreicht werden kann, die normalerweise Hexahydropyrazinderivat-haltige Formulierungen wegen ihres bitteren Geschmacks ablehnen.

Für die Herstellung des erfindungsgemäßen Granulats ist es ausreichend, wenn man Wirkstoff und Lösungsmittel getrennt oder eine Wirkstofflösung mit dem hydrophoben Trägerstoff zu einem wirkstoffhaltigen Granulat vermischt. Weiter war es überraschend, daß das so hergestellte Granulat auch von geschmacksempfindlichen Tieren ohne Zögern oral aufgenommen wird.

Das Granulat besteht aus den Komponenten:
- hydrophober Trägerstoff 80 bis 99,9 Gew.-%,
- Hexahydropyrazinderivate 0,1 bis 20 Gew.-%,
- gegebenenfalls weitere Hilfsstoffe.

Hexahydropyrazinderivate sind bekannt aus US-P 40 01 411, EP-A 13498, EP-A 185 012. Auf die dort angegebenen Strukturformeln und aufgeführten Einzelverbindungen wird hier besonders bezug genommen

Besonders hervorgehoben seien:
Praziquantel (2-Cyclohexylcarbonyl)-1,3,3,6,7-11b-hexahydro-4H-pyrazino[2,1-a]-isoquinolin-4-on und
Epsiprantel 2-(Cyclohexylcarbonyl)-2,3,6,7,8,12b-hexahydro-pyrazino[2,1-a]benzazepin-3(1H)-on.

Als hydrophobe Trägersubstanzen seien genannt Celluloseester wie Cellulosetriacetat, Cellulose-2,5-acetat, Cellulosepropionat, Cellulosebutyrat, hydrophobierte Kieselgele wie z.B. die in der Säulenchromatographie verwendeeten Reveresed Phase Phasen RP2(-dimethyl), RP4(-butyl), RP8(-octyl), RP18(-ocetadecyl), RP-Phenyl, RP-Nitril, Talkum, Bentionit und Dimethyldioctylammoniumbentionit (Bentionit 34).

Bevorzugt ist Celluloseacetat.

Den erfindungsgemäßen Granulaten können weitere anthelminthische Wirkstoffe zur Anwendung in einer Aufwandmenge pro kg von 0,1 bis 20 mg, bevorzugt 1 bis 10 mg, besonders bevorzut ca. 5 mg, zugesetzt werden.

Als solche Wirkstoffe seien genannt Phenylguanidine wie Febantel oder Netobimin; Benzimidazole, wie Fenbendazol, Albendazol, Oxibendazol, Oxfendazol, Mebendazol, Tricabendazol, Mebendazol, Fenbendazol, Parbendazol, Luxabendazol; Tetrahydropyrimidine wie Pyrantel, Morantel, Oxantel; Ivermectine und Avermectine wie Ivermectin, Abamectin, Moxidectin, Doramectin; Milbemycine; Levamisol, Tetramisol; cyclische Depsipeptide wie PF 1022.

Zur Herstellung der erfindungsgemäßen Granulate können auch weitere Hilfsstoffe wie Konservierungsstoffe, Antioxidantien, Lichtschutzmittel, Farbstoffe, resorptionsfördernde Stoffe, zerfallsfordernde Mittel, Bindemittel oder Schmiermittel, Stabilisatoren zugesetzt werden.

Als Konservierungsmittel seien genannt, z.B. Benzylalkohol, Benzoesäure, p-Hydroxybenzoesäure, Propionsäure und ihre Derivate und Salze, Sorbinsäure (-derivate und Salze).

Als Antioxidantien seien genannt z.B. Albumine; Aminosäuren, Ascorbinsäure, deren Salze und Derivate; Butylhydroxysanisol; Butylhydroxytoluol; derivatisierte Hydrochinone.

Als Lichtschutzfaktoren seien genannt z.B. Derivate von Aromaten, Stoffe mit einer geeigneten Absorptionswellenlänge.

Als Farbstoffe seien genannt z.B. Pigment, wie z.B. Eisenpigmente, Farbstoffe, die sich in wäßrigen Lösungen lösen oder solche, die sich im organischen lösen.

Als resorptionsfördernde Stoffe seien genannt z.B. Fettsäure, Fettsäureester und deren Gemische, Fettalkohole, Lecithin, Gallensäuresalze.

Als Stabilisatoren seien genannt z.B. Natriumsulfit, EDTA und dessen Salze.

Als Schmiermittel seien genannt z.B. Magnesiumstearat, Stearinsäure, Talkum, Bentonite; zerfallsfördernde Substanzen sind z.B. Stärke oder quervernetztes Polyvinylpyrrolidon, Bindemittel sind z.B. Stärke, Gelatine oder lineares Polyvinylpyrrolidon sowie Trockenbindemittel wie mikrokristalline Cellulose.

Das erfindungsgemäße Verfahren wird durchgeführt, indem die Wirkstoffe und Träger in Gegenwart von Lösungsmitteln gemischt werden. Dabei spielt die Reihenfolge des Zusammengebens der Komponenten keine Rolle. Die eingesetzten Lösungsmittel werden in der Regel bei der Granulat-Herstellung wieder verdampft.

So können z.B. die Komponenten Trägerstoff und Hilfsstoff in einer üblichen Mischapparatur vorgelegt und gemischt werden. Dieser Mischung wird Wirkstoff in Form seiner Lösung zugesetzt und gemischt.

Als Lösungsmittel für die Wirkstoffe seien genannt:

Ein- oder mehrwertige Alkohole wie Methanol, Propylenglykol, Ethanol, Isopropylalkohol, Ketone wie Aceton, aromatische und nicht-aromatische Kohlenwasserstoffe wie Toluol, Xylol, Ligroin, Essigsäureethylester, Wasser THF, Methylenchlorid, Dioxan.

Die Konzentration der Wirkstoff(salz)lösung liegt bei 0,5 bis 50 %; bevorzugt 10 bis 40 %

Die Einzelkomponenten können in Mischern jeglicher Bauart gemischt werden Um eine homogene Mischung zu erhalten, eignen sich z.B besonders Intensiv-Mischer mit Zerhackwerkzeugen. Die verwendeten Lösungen oder Wasser werden der trockenen Mischung in beliebiger Reihenfolge, auch alternierend, kontinuierlich oder diskontinuierlich durch Kippen, Schütten, Sprühen oder Vernebeln beigefügt.

Die feuchte Mischung wird weiterverarbeitet und dabei z.B. geraspelt, getrocknet, danach z.B. gesiebt oder mikronisiert.

Auch die Granulation im Wirbelschichtverfahren ist ein geeignetes Herstellverfahren. Hierbei werden z.B. die Lösungen über eine oder mehrere Düsen auf die sich bewegende Mischung aufgesprüht und gegebenenfalls dabei getrocknet.

Werden besonders kleine Partikel benötigt, ist gegebenenfalls auch ein Mikronisieren angezeigt (z.B. mit Hilfe einer Luftstrahl-, Perl- oder Pulvermühle).

Die erfindungsgemäß hergestellten Granulate können mit weiteren Trägerstoffen versetzt werden, hierzu dienen bei Futterapplikationen z.B. Einzelfuttermittel oder Gemische derselben. Solche Formulierungen können in Pulverform trocken oder angefeuchtet, extrudiert oder pelletiert werden. Sie können auch auf Futterpellets trocken aufgezogen werden. Eine Zugabe eines Bindemittels ist gegebenenfalls angezeigt. Solche Bindemittel sind z.B. pflanzliche, tierische oder synthetische Öle, Fette, Fettsäuren, -alkohole, Wachse, Gelatine. Granulate, hergestellt nach dem erfindungsgemaßen Verfahren können auch in feuchte Pellets eingearbeitet werden Solche Pellets können aus tierischen Materialien bestehen (z.B. moist pellet).

Die erfindungsgemäß hergestellten Granulate können u.a. auch in Kapseln gefüllt werden, die Kapselwand kann dabei aus Hart- oder Weichgelatine bestehen. Die Kapsel kann gegebenenfalls mit magensaftresistenten Überzügen behandelt sein.

Aus den erfindungsgemäß hergestellten Granulaten konnen auch andere oral applizierbare Formulierungen hergestellt werden, wie
- orale Lösungen, Konzentrate zur oralen Verabreichung nach Verdünnung;
- Emulsionen und Suspensionen zur oralen Anwendung;
- Pasten bzw. Zubereitungen, bei denen die Formulierung in einer Salbengrundlage oder in einer Öl in Wasser oder Wasser in Öl Emulsionsgrundlage verarbeitet ist;
- Feste Zubereitungen wie Pulver, Premixe oder Konzentrate, Pellets, Extrudate, Tabletten, Boli, Kapseln;
- oder Kombinationen der erwähnten Formen.

Die erfindungsgemäßen Formulierungen eignen sich bei günstiger Warmblütertoxizität insbesondere zur Bekämpfung von pathogenen Endoparasiten die bei Haustieren wie z.B. Katzen und Hunden vorkommen. Sie sind dabei gegen alle oder einzelne Entwicklungsstadien der Schädlinge sowie gegen resistente und normal sensible Arten wirksam. Zu den pathogenen Endoparasiten zählen Cestoden, Trematoden, Nematoden, Acantocephalen, insbesondere:
Aus der Ordnung der Pseudophyllidea z.B.: Diphyllobothrium spp., Spirometra spp., Schistocephalus spp..
Aus der Ordnung der Cyclophyllidea z.B.: Mesocestoides spp., Anoplocephala spp., Paranoplocephala spp., Moniezia spp., Taenia spp., Echinococcus spp., Hydatigera spp., Diorchis spp., Dipylidium spp., Joyeuxiella spp., Spyrometra spp..
Aus der Unterklasse der Digenea z.B.: Schistosoma spp., Fasciola spp., Dicrocoelium spp., Opisthorchis spp..
Aus der Ordnung der Enoplida z.B.. Trichuris spp, Capillaria spp., Trichinella spp..
Aus der Ordnung der Rhabditia z.B.: Micronema spp., Strongyloides spp..
Aus der Ordnung der Strongylida z.B : Stronylus spp., Triodontophorus spp., Oesophagodontus spp., Trichonema spp., Gyalocephalus spp., Poteriostomum spp., Cyclicocyclus spp., Stephanurus spp., Ancyclostoma spp., Uncinaria spp., Cyathostomum spp., Metastrongylus spp., Dictyocaulus spp., Muellerius spp., Protostrongylus spp., Elaphostrongylus spp., Parelaphostrongylus spp., Crenosoma spp., Paracrenosoma spp., Filaroides spp., Parafilaroides spp., Marshallagia spp., Hyostrongylus spp., Ollulanus spp., Craterostomum spp., Cyclicodontophorus spp., Hyalocephalus spp., Cylindropharynx spp., Caballonema spp., Elaeophorus spp., Dirofilaria spp., Onchocerca spp., Setaria spp..
Aus der Ordnung der Oxyurida z.B.: Oxyuris spp., Enterobius spp..
Aus der Ordnung der Ascaridia z.B. Ascaris spp., Toxascaris spp., Toxocara spp., Parascaris spp., Probstmangria spp..
Aus der Ordnung der Spirurida z.B.. Thelazia spp., Habronema spp., Draschia spp., Dracunculus spp..

Die erfindungsgemäßen Mittel eignen sich außerdem bei günstiger Warmblütertoxizität zur Bekämpfung von pathogenen Endoparasiten die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B Nerze, Chinchilla, Waschbär, Vögel wie z.B. Hühner, Gänse, Puten, Enten, Strauß.

Zu den Fischen gehören Nutz-, Zucht-, Aquarien- und Zierfische aller Altersstufen die in Süß-, Salz- und Brackwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, japanese yellowtail (Seriola quinqueradiata), Japanaal (Anquilla japonica), Red seabream (Pagurus major), Seabass (Dicentrarchus labrax), Grey mullet (Mugilus cephalus), Pompano, Gilthread seabream (Sparus auratus), Tilapia ssp., Chichliden-Arten wie z.B. Plagioscion, Channel catfish.

Zu den pathogenen Endoparasiten zählen Cestoden, Trematoden, Nematoden, Acantocephalen insbesondere:
Aus der Ordnung der Pseudophyllidea z.B.: Diphyllobothrium spp., Spirometra spp., Schistocephalus spp., Ligula spp., Bothridium spp, Diphlogonoporus spp..
Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp, Dipylidium spp., Joyeuxiella spp., Diplopylidium spp..
Aus der Unterklasse der Monogenea z.B.: Gyrodactylus spp., Dactylogyrus spp., Polystoma spp..
Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp, Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp , Opisthorchis spp., Clonorchis spp. Metorchis spp, Heterophyes spp., Metagonimus spp..
Aus der Ordnung der Enoplida z.B.: Trichuris spp, Capillaria spp., Trichomosoides spp., Trichinella spp..
Aus der Ordnung der Rhabditia z.B.: Micronema spp., Strongyloides spp..
Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp , Cylicostephanus spp., Oesophagostomum spp., Chabertia spp, Stephanurus spp., Ancylostoma spp., Uncinaria spp., Bunostomum spp.,
Globocephalus spp., Syngamus spp., Cyathostoma spp, Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..
Aus der Ordnung der Oxyurida z.B.: Oxyuris spp, Enterobius spp., Passalurus spp., Syphacia spp., Aspiculuris spp., Heterakis spp..
Aus der Ordnung der Ascaridia z.B.: Ascaris spp., Toxascaris spp., Toxocara spp., Parascaris spp., Anisakis spp., Ascaridia spp..
Aus der Ordnung der Spirurida z.B.: Gnathostoma spp., Physaloptera spp., Thelazia spp., Gongylonema spp., Habronema spp., Parabronema spp., Draschia spp., Dracunculus spp..
Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..
Aus der Ordnung der Gigantorhynchida z.B. Filicollis spp., Moniliformis spp., Macracanthorhynchus spp., Prosthenorchis spp..

Die Anwendung kann sowohl prophylaktisch als auch therapeutisch erfolgen

Die bevorzugte Anwendungskonzentration der Wirkstoffe in den erfindungsgemäßen Mischungen liegt pro kg Lebendgewicht bei 1 bis 300 mg, bevorzugt bei 5 bis 50 mg

### Beispiel 1

In einem Liter einer 10 %igen Praziquantellösung in Ethanol werden 90 g Cellulosetriacetat suspendiert und das Ethanol langsam verdampft. Man erhält 100 g eines 10 %igen Praziquantel-haltigen Pulvers.

## Patentansprüche

1. Verfahren zur Herstellung oral applizierbarer Formulierungen von Hexahydropyrazinderivaten, **dadurch gekennzeichnet, daß** man den Wirkstoff in Gegenwart geeigneter Lösungsmittel mit hydrophoben Trägerstoffen gegebenenfalls in Gegenwart von Hilfsstoffen vermischt und die erhaltene Mischung gegebenenfalls in weitere anwendungsfertige Formen überführt.

2. Granulat bestehend aus den Komponenten:
- hydrophober Trägerstoff 80 bis 99,9 Gew.-%,
- Hexahydropyrazinderivate 0,1 bis 20 Gew.-%,
- gegebenenfalls weitere Hilfsstoffe

## Claims

1. Process for preparing orally administrable formulations of hexahydropyrazine derivatives, **characterized in that** the active compound is mixed in the presence of suitable solvents with hydrophobic carriers, if appropriate in the presence of auxiliaries, and the resulting mixture is, if appropriate, converted into other ready-to-use forms.

2. Granules, comprising the components:
- hydrophobic carrier 80 to 99.9% by weight,
- hexahydropyrazine derivatives 0.1 to 20% by weight,
- if appropriate further auxiliaries.

## Revendications

1. Procédé de préparation de formulations administrables par voie orale, de dérivés d'hexahydropyrazine, **caractérisé en ce que** l'on mélange l'agent actif en présence d'un solvant approprié, avec des substances de support hydrophobes, le cas échéant en présence d'auxiliaires et on convertit le mélange obtenu, le cas échéant en d'autres formes prêtes à l'emploi.

2. Granules consistant en les composants :
- matière de support hydrophobe, 80 à 99,9% en poids ;
- dérivé d'hexahydropyrazine, 0,1 à 20% en poids, et
- le cas échéant, d'autres auxiliaires.
